# EUROPÄISCHE PATENTANMELDUNG

(11) **EP 2 359 898 A2**
(43) Veröffentlichungstag der Anmeldung: **24.08.2011**
(21) Anmeldenummer: 11153010.1
(22) Anmeldetag: 02.02.2011
(51) Int. Cl.: A61N 1/37, H05K 9/00

(54) **Implantierbares Element mit Mitteln zur Verringerung einer Flussdichte**

(30) Priorität: 24.03.2010 US 316876 P; 11.02.2010 DE 102010000373
(71) Anmelder: BIOTRONIK SE & Co. KG, 12359 Berlin (DE)
(72) Erfinder: Weiss, Ingo, 12435, Berlin (DE)
(74) Vertreter: Lindner-Vogt, Karin L.

(57) **Zusammenfassung**

Implantierbares Element mit einem langgestreckten Grundkörper, einem sich in Längsrichtung des Grundkörpers erstreckenden oder diesen bildenden Funktions-Leiter, der zur Realisierung einer medizinischen Funktion des Elementes wirkt und einen induktiven Abschnitt hat, und Magnetfluss-Erzeugungsmitteln zur Erzeugung eines magnetischen Flusses in der Umgebung des Funktions-Leiters, insbesondere von dessen induktivem Abschnitt, die magnetisch derart mit dem Funktions-Leiter koppeln, dass dem bei einem Stromfluss durch den Funktions-Leiter erzeugten magnetischen Fluss entgegen gewirkt und somit die Stromflussdichte durch den Funktions-Leiter verringert wird.

## Beschreibung

Die Erfindung betrifft ein implantierbares Element mit einem langgestreckten Grundkörper und einem sich in Längsrichtung des Grundkörpers erstreckenden Funktions-Leiter, der zur Realisierung einer medizinischen Funktion des Elements wirkt. Derartige Elemente sind insbesondere Stimulations-Elektrodenleitungen (mitunter verkürzt auch als "Elektroden" bezeichnet) von Herzschrittmachern oder Schockelektrodenleitungen von implantierbaren Defibrillatoren, aber auch Katheter, die eine langgestreckte leitfähige Struktur enthalten, oder auch Führungsdrähte oder Gefäßstützen (Stents), bei denen der Grundkörper selbst den Funktions-Leiter im Sinne der Terminologie dieser Patentanmeldung darstellt. Die Erfindung betrifft des Weiteren ein elektronisches Implantat, welches üblicherweise mit einer Leitung im Körper eingesetzt wird.

Medizinische Implantate wie die erwähnten Schrittmacher und Defibrillatoren besitzen häufig eine elektrische Verbindung zum Körperinneren des Patienten. Eine solche Verbindung dient zur Messung von elektrischen Signalen und/oder zur Stimulation von Körperzellen. Diese Verbindung ist of als längliche Elektrode ausgeführt. Gegenwärtig werden elektrische Signale zwischen dem Implantat und den Elektrodenkontakten (Spitze, Ringe, HV-Schockwendeln, Sensoren o. ä.) mit elektrisch gut leitenden Materialien übertragen.

Wird ein System aus Implantat und Elektrode starken Störfeldern (EMI, MRI) ausgesetzt, so kann es zu unerwünschtem Fehlverhalten kommen, speziell einer Erwärmung von Teilen des Systems oder elektrischen Fehlfunktionen (z. B. Resets).

Die Ursache für das unerwünschte Fehlverhalten ist die Wechselwirkung des Feldes mit der länglichen Leitungsstruktur, die eine Induktivität bildet: Diese wirkt als eine Antenne und empfängt Energie aus den umgebenden Feldern. Diese Energie auf den therapeutisch genutzten Leitungen kann die Antenne distal über die Elektrodenkontakte (Spitze, Ring...) an das Gewebe oder proximal an das Implantat abgeben.

Vergleichbare Probleme treten auch bei anderen länglichen leitfähigen Strukturen auf, deren proximales Ende nicht zwingend mit einem Implantat verbunden ist (z. B. bei Kathetern, Führungsdrähten, temporären Elektroden, Stents etc.).

In den letzten Jahren wurden bereits Gegenmaßnahmen zur Lösung der erwähnten Probleme vorgeschlagen. Neben Maßnahmen zur Abschirmung der RF-Energie von der Elektrode oder dem Einbringen eines Bandpassfilters in die Elektrodenzuleitung (vgl. US 7,363,090 B2) ist auch ein Thermoschalter in der Elektrodenzuleitung bekannt; vgl. US 2009/0105789. Ein solcher Thermoschalter hat neben ungünstigen konstruktiven Eigenschaften (Steifheit der Elektrode) auch die Nachteile einer deutlichen Hysterese und eines schlecht einstellbaren und kontrollierbaren Temperaturbereiches und kann die Elektrodenspitze nur komplett zu- oder abschalten, so dass im abgeschalteten Zustand keine Stimulation oder Ableitung von Signalen mehr möglich ist. Damit ist die Anwendung diese Elektrode für schrittmacherabhängige Patienten ausgeschlossen.

Die Lösungen aus Beispielsweise US 7,363,090 B2 erlauben es zwar, einen elektrisch durchgängigen Pfad für tiefe Frequenzen (d. h. für die herkömmliche Schrittmacherfunktion Pacing und Sensing) bereitzustellen, sie sind jedoch in ihrer bisher bekannten Ausführung steif und beeinträchtigen die Steuerbarkeit der Elektrode bei Implantation erheblich, so dass der klinische Nutzen de facto in Frage gestellt ist. Ebenso erfordern diese bekannten Lösungsvarianten zusätzliche, im herkömmlichen Produktionsprozess einer Elektrode nicht erforderliche, Kontaktierungsstellen, um diese als diskretes Element implementierte Lösung einzubauen. Die bringt zusätzlich Risikostellen in das Design einer solchen Elektrode ein.

Weitere Ansätze zur Lösung der oben beschriebenen Probleme sind in der US 2009/0198314, die eine geeignete Dimensionierung und einen koaxial mehradrigen Aufbau einer Schrittmacherelektrodenleitung offenbart, sowie in der US 2009/0171421 beschrieben, die den Einbau einer Mehrzahl voneinander beabstandeter Schaltungssegmente mit hoher Impedanz in eine Elektrodenleitung mit gewendeltem Funktions-Leiter lehrt. Außerdem wird hingewiesen auf die WO 2009/049310 A1, in der als Anordnung zur Unterdrückung von Wechselstromausbreitung längs langgestreckter Implantate ebenfalls mehradrige Leiteranordnungen mit koaxialem Aufbau oder in Parallelanordnung langgestreckter Leiter vorgeschlagen werden.

Der Erfindung liegt die Aufgabe zugrunde, eine verbesserte implantierbare Leitung der genannten Art bereit zu stellen, die in starken äußeren Feldern verbesserte Eigenschaften aufweist. Des Weiteren soll ein elektronisches Implantat mit verringerter Störanfälligkeit in äußeren Feldern bereitgestellt werden.

Diese Aufgabe wird durch ein implantierbares Element mit den Merkmalen des Anspruchs 1 oder 2 bzw. ein elektronisches Implantat mit den Merkmalen des Anspruchs 15 gelöst. Zweckmäßige Fortbildungen des Erfindungsgedankens sind Gegenstand der abhängigen Ansprüche.

In einer Ausführung der Erfindung ist vorgesehen, dass die Magnetfluss-Erzeugungsmittel einen induktiv wirkenden Leiterabschnitt, der ohne Unterbrechung des Verlaufes des Funktions-Leiters in den Grundkörper eingefügt oder auf diesen aufgesetzt ist, und ein hieran zur Bildung eines LC-Gliedes angeschlossenes kapazitives Element aufweisen.

In einer weiteren Ausführung ist das implantierbare Element ausgebildet als Elektrodenleitung mit mindestens einer Elektrode und einem Elektrodenanschlusskontakt an einem Ende der Leitung, wobei der Funktions-Leiter die Elektrode mit dem Elektrodenanschlusskontakt verbindet und mindestens einen gewendelten Abschnitt aufweist und die Stromfluss-Erzeugungsmittel einen koaxial oder koradial mit dem gewendelten Abschnitt des Funktions-Leiters gewendelten Flusserzeugungsabschnitt aufweisen.

In einer hierzu alternativen Ausführung ist das implantierbare Element ausgebildet als Führungsdraht oder Katheter oder Gefäßstütze, wobei der Funktions-Leiter insbesondere mindestens einen gewendelten Abschnitt aufweist und die Stromfluss-Erzeugungsmittel einen koaxial oder koradial mit dem gewendelten Abschnitt des Funktions-Leiters gewendelten Flusserzeugungsabschnitt aufweisen.

Eine Ausgestaltung beider Varianten sieht vor, dass die Magnetfluss-Erzeugungsmittel mehrere längs des Funktions-Leiters hintereinander geschalteter Flusserzeugungsabschnitte umfassen.

In einer weiteren Ausführung der Erfindung ist vorgesehen, dass die Magnetfluss-Erzeugungsmittel ein in einen Innenraum des induktiven Abschnitts des Funktions-Leiters eingebettetes oder den induktiven Abschnitt mindestens teilweise umgebendes kapazitives Element aufweisen. Hierbei ist insbesondere das in den induktiven Abschnitt eingebettete oder ihn mindestens teilweise umgebende kapazitive Element durch zwei koaxiale Metallzylinder mit der dielektrischen Füllung gebildet. Noch spezieller ist vorgesehen, dass längs des Funktions-Leiters mehrere kapazitive Elemente hintereinander angeordnet sind.

Eine weitere Ausführung des erfindungsgemäßen implantierbaren Elementes zeichnet sich dadurch aus, dass die Magnetfluss-Erzeugungsmittel mehrere Komponenten aufweisen, die durch Bemessung der jeweiligen Induktivität und/oder Kapazität auf verschiedene Frequenzen eines äußeren Feldes abgestimmt sind. Insbesondere sind hierbei die auf verschiedene Frequenzen abgestimmten Komponenten der Magnetfluss-Erzeugungsmittel durch längs des Funktions-Leiters hintereinander angeordnete induktive Abschnitte mit eingebetteten oder umgebenden kapazitiven Elementen gebildet, die verschiedene Werte der Induktivität bzw. Kapazität haben.

Eine weitere Ausführung ist derart ausgestaltet, dass der oder mindestens ein Flusserzeugungsabschnitt der Magnetfluss-Erzeugungsmittel als leitfähige Beschichtung des Funktions-Leiters, insbesondere von dessen induktiven Abschnitt, ausgebildet ist.

In einer weiteren Ausführung ist das implantierbare Element mit mechanischen Verstellmitteln zur Veränderung der Relativposition zwischen dem induktiven Abschnitt des Funktions-Leiters und dem oder einem Flusserzeugungsabschnitt der Magnetfluss-Erzeugungsmittel versehen.

Eine vorteilhaft auf verschiedene Anwendungssituationen anpassbare Ausgestaltung zeichnet sich dadurch aus, dass die Magnetfluss-Erzeugungsmittel, insbesondere im implantierten Zustand des Elementes, auf eine vorbestimmte Wirkfrequenz einstellbar sind.

Die erfindungsgemäßen Mittel können in eine Elektrode eingebaut werden und ihre Funktion ausüben, ohne dass der zu beeinflussende Leiter unterbrochen werden muss. Die Zuverlässigkeit einer so konstruierten Elektrode ist nicht durch zusätzliche Kontaktstellen (wie im Falle bekannter Lösungen) beeinträchtigt.

Die Mittel können, bei geeigneter Ausgestaltung, so in die Elektrodenleitung integriert werden, dass ein koaxiales Innenlumen bleibt, durch das ein Mandrin durchfahren kann. Damit bleibt (im Gegensatz zu einer bekannten Lösung) die Steuerbarkeit einer solchen Elektrode unbeeinträchtigt.

Die Mittel können in Segmente unterteilt werden. Dadurch entsteht kein längerer steifer Abschnitt.

Die Mittel können unter Zuhilfenahme der ohnehin koaxial liegenden Außenwendel realisiert werden, zumindest kann diese dazu abschnittsweise genutzt werden.

Ein Ausfall der erfindungsgemäßen Mittel hat nur den Verlust der MR-Sicherheit zu Folge, und kompromittiert nicht den therapeutischen Zweck, z. B. als Stimulationselektrode.

Die Lösung ist auch auf Elektroden mit nicht gewendelten Leitern übertragbar, indem deren Leiter (z. B. Seile) nur abschnittsweise zu einer Helix geformt werden (induktiver Abschnitt), ohne sie aber zu unterbrechen.

Die erfindungsgemäßen Mittel lassen sich so realisieren, dass sie nachträglich auf Standardelektroden montiert werden können (d. h. diese nachgerüstet werden können).

In speziellen Ausführungen der Erfindung sind folgende Merkmale vorgesehen:
1. Der induktiv zu beeinflussende Leiter ist ein Leiter innerhalb des Implantatsgehäuses/Elektronik.
2. Der induktiv zu beeinflussende Funktions-Leiter ist ein (langer > 5 cm) Stent.
3. Der induktiv zu beeinflussende Leiter ist ein (langes > 5 cm) orthopädisches Implantat (elektrisch leitfähig).
4. Der induktiv zu beeinflussende Leiter befindet sich außerhalb des Implantatsgehäuses, insbesondere ist dieser eine Elektrodenzuleitung eines IPGs.
5. Die Vorrichtung zur Erzeugung des magnetischen Flusses (bzw. Gegenflusses) ist ein Schwingkreis.
6. Die Güte des Schwingkreises ist dahingehend eingestellt, dass der Impedanzbetrag des Leiterelementes, dessen magnetischer Fluss dadurch beeinflusst wird, mehr als 200'Ω beträgt.
7. Der Schwingkreis ist im Speziellen ein passiver LC-Schwingkreis; allgemein besteht er aus einem induktiven und kapazitiven Element, das jeweils auch durch räumlich verteilte Materialeigenschaften realisiert werden kann.
8. Der Schwingkreis ist ein Parallelschwingkreis.
9. Das induktive Element des Schwingkreises koppelt magnetisch mit dem genannten elektrischen Leiter, insbesondere mit dessen induktivem Abschnitt (bzw. Element).
10. Durch geometrische Formung des genannten elektrischen Leiters selbst wird zumindest lokal ein ausgeprägt induktiver Abschnitt realisiert, besonders dazu ausgelegt, um mit dem induktiven Element des Schwingkreises zu koppeln.
11. Der induktive Abschnitt wird durch eine in die Leitung seriell eingesetzte Spule bzw. Primärspule eines Transformators realisiert.
12. Der induktive Abschnitt wird durch geometrische Formung des Leiters, insbesondere in Helixform oder Planarspulenform, erzeugt, ohne dass der Leiter unterbrochen und ein zusätzliches Bauelement mittels Kontaktierungstechnik zwischengeschaltet wird.
13. Das induktive Element des Schwingkreises ist das geometrische Pendant des induktiven Leiterabschnittes, im speziellen die Sekundärspule des Transformators, da dass maximale Kopplung erzeugt wird. Der Kopplungsfaktor ist bevorzugt k>0.5.
14. Die Kopplungsrichtung ist derart, dass der Stromfluss im genannten elektrischen Leiter reduziert wird.
15. Die Resonanzfrequenz des Schwingkreises ist darauf abgestimmt, dass Ströme in genannten elektrischen Leitern nur selektiv hinsichtlich der gewünschten Frequenz unterdrückt werden.
16. Eine Anwendung sieht vor, insbesondere engbandige Störsignale innerhalb einer Bandbreite von < 10 MHz zu unterdrücken.
17. Das induktive Element/Abschnitt ist eine Solenoidspule, die koaxial oder koradial mit der (in diesem Fall gewendelten bzw. zu diesem Zweck lokal gewendelten Elektrodenzuleitung) angeordnet ist.
18. Das induktive Element/Abschnitt ist eine Flachspule.
19. Die induktiven Elemente/Abschnitte sind mechanisch flexibel realisiert.
20. Die induktiven Elemente/Abschnitte erlauben eine Kavität (die Vorrichtung kann so in die Elektrodenleitung integriert werden, dass ein koaxiales Innenlumen bleibt durch das ein Mandrin durchfahren kann. Damit bleibt die Steuerbarkeit einer solchen Elektrode unbeeinträchtigt).
21. Der Kondensator des Schwingkreises ist koaxial um das induktive Element realisiert.
22. Der Kondensator ist mechanisch flexibel.
23. Das induktive Element/Abschnitt (insbesondere das des Schwingkreises) hat einen Reihenwiderstand r<0.05'Ω pro Windung.
24. Der Kondensator (das kapazitive Element) hat einen ESR< 0.05 'Ω, das verwendete Dielektrikum hat eine relative Permittivität >8.
25. Der Schwingkreis kann auch mindestens einen galvanischen Kontakt zum genannten elektrischen Leiter haben, ohne aber diesen unterbrechen zu müssen.
26. Die erfindungsgemäßen Mittel können mehrfach hintereinander geschaltet werden und über die Leiterlänge verteilt sein.
27. Die zu unterdrückenden Frequenzen sind >1 MHz.
28. Mittel können für unterschiedliche Frequenzen realisiert sein und innerhalb desselben Implantats/Leiters hintereinander geschaltet werden, so dass gleichzeitig Schutz gegen alle diese Frequenzen besteht.
29. Das induktive Element des Schwingkreises ist zwischen die Wicklungen einer gewendelten Leitung zwischengewickelt (koaxial oder koradial).
30. Das induktive Element des Schwingkreises ist als eine hoch leitfähige Beschichtung auf dem induktiven Element des genannten Leiters realisiert, im Speziellen als Draht um den Leiter gewendelt (der an sich gewendelt sein kann - wie bei Musikinstrumentsaiten).
31. Das induktive Element des Leiters ist ein Abschnitt der gewendelten Elektrodenzuleitung selbst.
32. Die Länge des induktiven Elements ist <= 1 cm.
33. Die induktiven Elemente sind aus mehradriger Litze realisiert.
34. Das kapazitive Element wird durch ein dielektrisches Material realisiert, das zwischen den Windungen des induktiven Elementes eingebracht ist.
35. Die erfindungsgemäßen Mittel sind als nachträglich montierbares Element auf eine herkömmliche Leitung (insbesondere IPG Elektrode) realisiert (Standardelektroden sind dadurch nachrüstbar).
36. Die erfindungsgemäßen Mittel sind unter Zuhilfenahme der koaxial liegenden Au-βenwendel (abschnittsweise) einer multipolaren Elektrode realisiert.
37. Die Mittel (als elektrisch aktive Vorrichtung realisiert) speisen ein generiertes Signal ein, um den Gegenfluss zu erzeugen; das Implantat/die Elektrode ist mit einem entsprechenden Generator ausgerüstet.
38. Der Schwingkreis enthält aktive (batteriebetriebene) Elemente zur Erhöhung der Güte.
39. Die beiden Spulen eines zwischen der Funktions-Wendel und der zusätzlichen Induktivität gebildetes Übertragers (L, L1) sind mechanisch mobil, so dass L mechanisch einen Steigungsgeber realisiert.
40. Der Abschnitt von L1 ist mindestens über die Länge der möglichen Translationsbewegung mit einer elektrisch hochleitenden Schicht überzogen, wobei sich die Windungen in diesem Abschnitt aber nicht untereinander berühren.
41. L (Schraube) wird koaxial über L1 (Mutter) realisiert, so dass eine Schraube-Mutter-Verbindung realisiert wird.
42. Zwischen den Windungen der Schraube und der Mutter kann galvanischer Kontakt bestehen, dieser ist beweglich im Sinne eines Schleifkontaktes.
43. Über die Translationslänge sind sowohl die Windungen der Schraube (L1) als auch die Rillen der Mutter (L) elektrisch hochleitend. Die Leitfähigkeit dieser Beschichtung ist bevorzugt mehr als 2x höher als die der Wendel (Elektrodenzuleitung).
44. Die Mittel sind hinsichtlich der Resonanzfrequenz (Wirkfrequenz der Signalsperrung) justierbar.
45. Die Justierung kann im implantierten Zustand erfolgen.
46. Die Justierung erfolgt durch Trimmung des Kondensators (z. B. spannungsabhängige Kapazität).
47. Die Justierung erfolgt automatisch in Abhängigkeit der Störfrequenz (d. h. das Implantat passt die Schutzwirkung automatisch an die Arbeitsfrequenz z. B. eines MRI-Scanners an).
48. Die Justierung erfolgt über ein Signal, das vom Implantat erzeugt wird.
49. Das Implantat ist von extern programmierbar, ein solches Signal zu erzeugen.

Vorteile und Zweckmäßigkeiten der Erfindung ergeben sich im Übrigen aus der nachfolgenden Beschreibung von speziellen Ausführungen bzw. von deren Berechnungsgrundlagen anhand der Figuren. Von diesen zeigen:
- Fig. 1: ein Ersatzschaltbild zur Erläuterung einer ersten Ausführung der Erfindung mit zugehörigen Berechnungsgrundlagen,
- Fig. 2: ein Ersatzschaltbild zur Erläuterung einer ersten Ausführung der Erfindung mit zugehörigen Berechnungsgrundlagen,
- Fig. 3: eine graphische Darstellung zur Verdeutlichung des Impedanzanstieges, den ein induktives Element bei einer Ausführungsform der Erfindung durch induktive Kopplung der Magnetflusssperre hervorbringt, mit zugehörigen Berechnungsparametern,
- Fig. 4A und 4B: skizzenartige Darstellungen der Leiterstruktur einer erfindungsgemäßen Leitung (Fig. 4B mit Realisierungsbeispiel eines KondensatorBauelement),
- Fig. 5: eine skizzenartige Darstellung der Leiterstruktur einer weiteren erfindungsgemäßen Leitung,
- Fig. 6: eine skizzenartige Darstellung der Leiterstruktur einer weiteren erfindungsgemäßen Leitung,
- Fig. 7: eine skizzenartige Darstellung der Leiterstruktur einer weiteren erfindungsgemäßen Leitung,
- Fig. 8: eine skizzenartige Darstellung der Leiterstruktur einer weiteren erfindungsgemäßen Leitung,
- Fig. 9: eine skizzenartige Darstellung der Leiterstruktur einer weiteren erfindungsgemäßen Leitung,
- Fig. 10: eine skizzenartige Darstellung der Leiterstruktur einer weiteren erfindungsgemäßen Leitung,
- Fig. 11: eine skizzenartige Darstellung der Leiterstruktur einer weiteren erfindungsgemäßen Leitung,
- Fig. 12: eine skizzenartige Darstellung des Wirkprinzips einer weiteren Ausführungsform,
- Fig. 13: eine skizzenartige Darstellung des Wirkprinzips einer weiteren Ausführungsform,
- Fig. 14: ein Ersatzschaltbild zur Erläuterung der letztgenannten Ausführung.

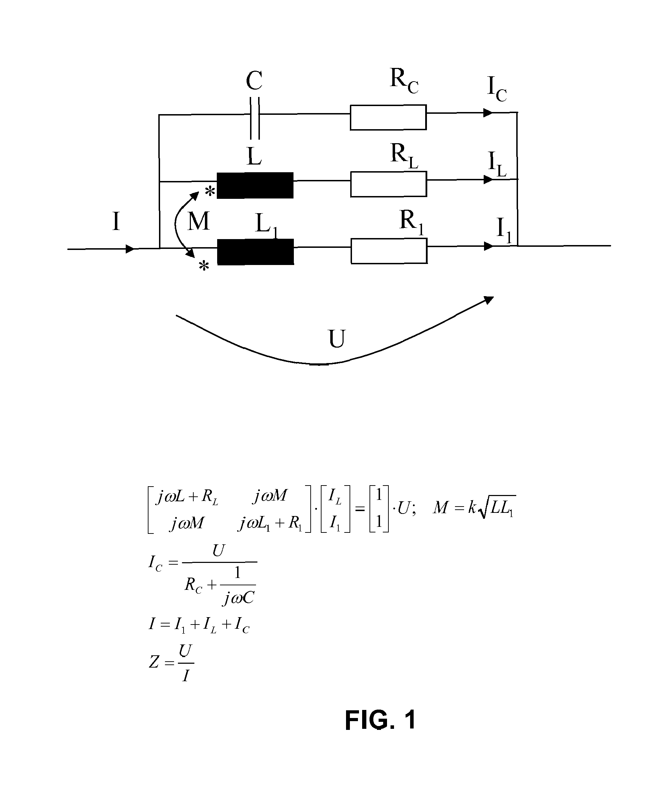

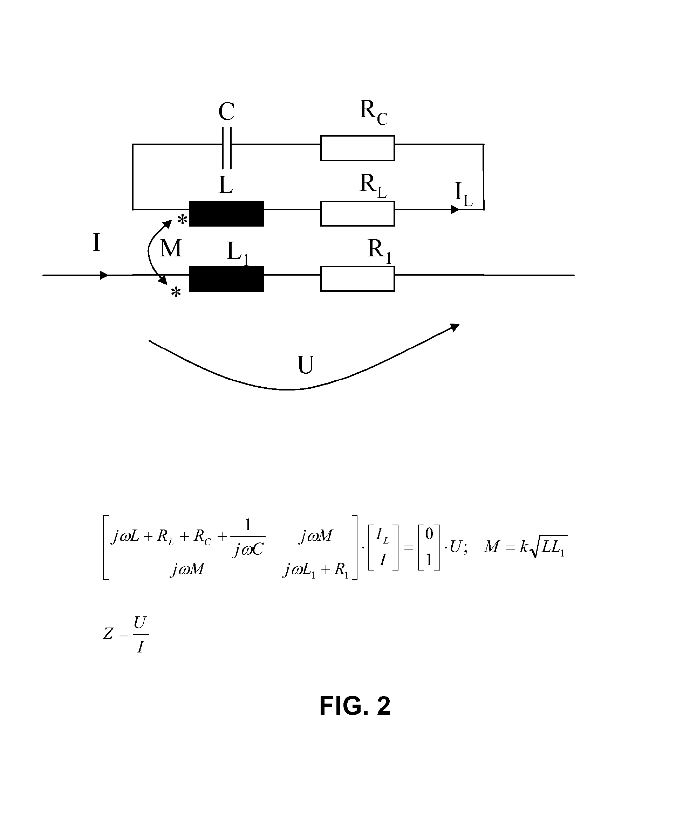

Fig. 1 und 2 zeigen, jeweils in Form eines Ersatzschaltbildes mit zugehörigen Berechnungsgrundlagen, Darstellungen zur Erläuterung des erfindungsgemäßen Prinzips in zwei Ausführungen. R1 und L1 bezeichnen dabei einen ohmschen Widerstand bzw. eine Induktivität eines Funktions-Leiters, und L und C bezeichnen die Induktivität bzw. Kapazität sowie RL und RC die entsprechenden Verlustwiderstände eines LC- Schwingkreises, welcher als Magnetfluss-Erzeugungsmittel zum Aufbau eines magnetischen Flusses dient, der zur Verringerung des durch ein äußeres Feld im Funktions-Leiter aufgebauten Stromflusses in diesen eingekoppelt wird. Der LC-Schwingkreis wird auf der Grundlage der in den Figuren angegebenen Gleichungen auf die im Implantat zu unterdrückende Feldfrequenz (bzw. ein Frequenzband) abgestimmt, um den Betrag des Magnetflusses (Gegenflusses) zu maximieren.

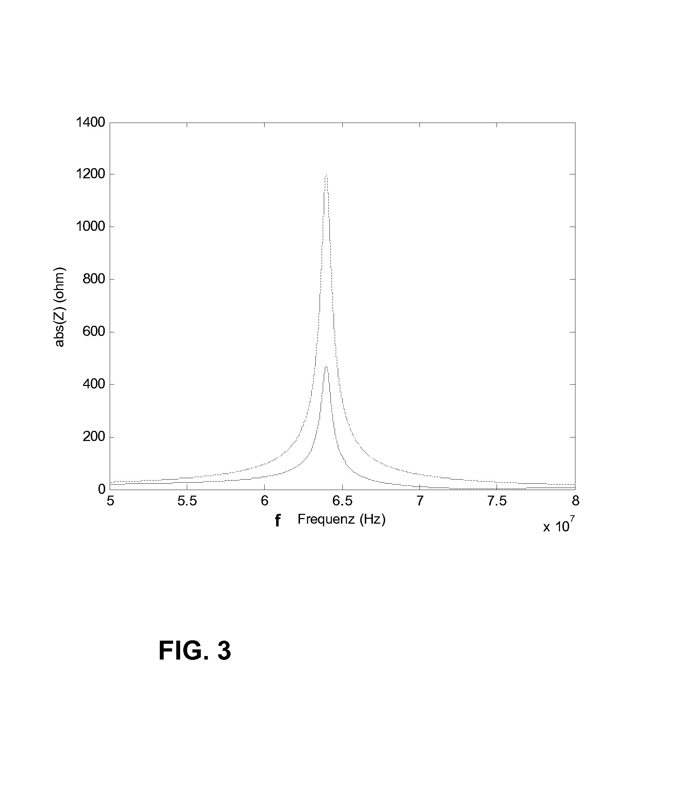

Fig. 3 zeigt die Ergebnisse einer Berechnung (Simulation) des frequenzabhängigen Impedanzanstieges, den der induktive Abschnitt des Funktions-Leiters allein auf Grund der induktiven Kopplung mit den Magnetfluss-Erzeugungsmitteln (der "Magnetflusssperre") hervorbringt. Die Berechnungsparameter sind dabei:

| | |
|---|---|
| r=.5e-3; | % Wendelradius (m) |
| 1=.5e-2; | % Spulenlänge (m) |
| n=3; | % Windungen pro m |
| Rc=.02; | % ESR des Caps ESR=.02ohm@64MHz |
| r1=.01; | %Widerstand Resonatorspule pro Windung (ohm) |
| r11=30/.6; | % Wendelwiderstand pro m Wendel (ohm) |
| k=.8; | % Kopplungsfaktor (Kurve mit höheren Maximum) |
| k=.5; | % Kopplungsfaktor (Kurve mit tieferem Maximum) |

Berechnet wird hieraus gemäß Fig. 1 oder Fig. 2 der erforderliche Kapazitätswert C.

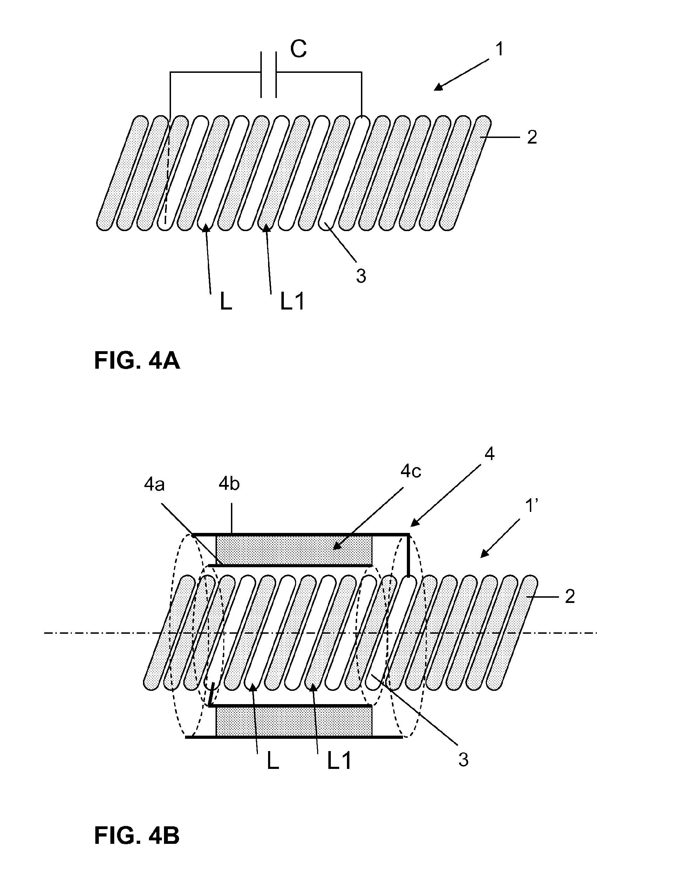

Fig. 4A zeigt schematisch den konstruktiven Aufbau des Inneren einer Elektrodenleitung 1, die einen gewendelten Funktions-Leiter 2 umfasst, der in einem Abschnitt eine Induktivität L1 hat und in den koradial, mit gleichem Durchmesser und gleicher Steigung, ein weiterer Leiter 3 mit einer Induktivität L gewickelt ist, welchem eine Kapazität C zugeordnet ist. Fig. 4B zeigt, dass die Kapazität C durch einen die Elektrodenwendel 2 bzw. 3 umgebenden Kondensator 4 realisiert sein kann, der aus zwei konzentrischen Metallzylindern 4a, 4b mit einem dazwischen angeordneten Dielektrikum 4c aufgebaut ist. Um die bei typischen Störfeldern erforderliche Kapazität im pF-Bereich zu erreichen, können im Hinblick auf realistische Abmessungen des Magnetfluss-Erzeugungsabschnittes Dielektrika mit einer relativen Dielektrizitätskonstante (DK )> 8 eingesetzt werden.

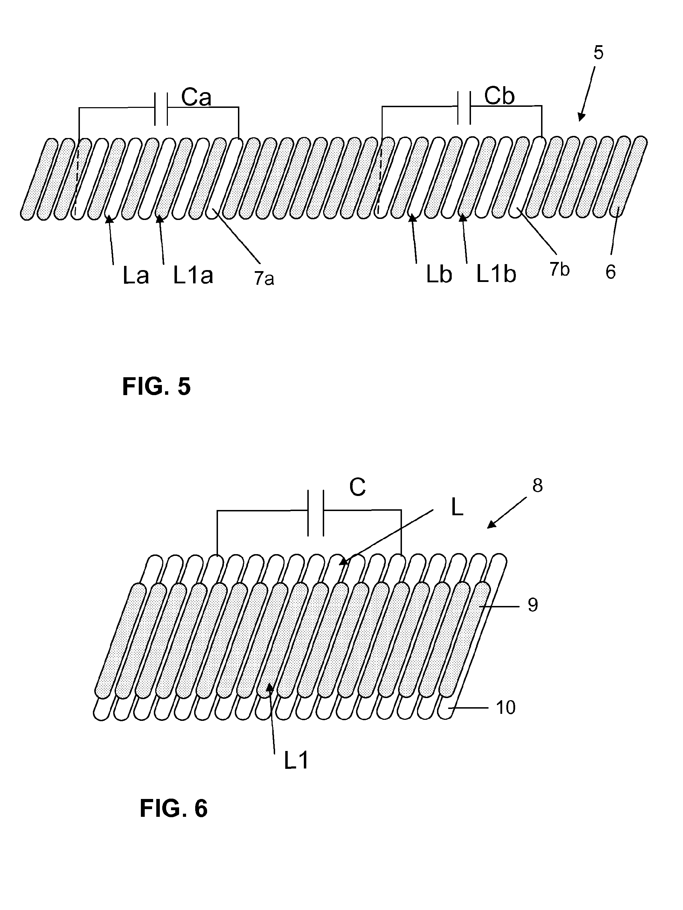

Fig. 5 zeigt als weitere Ausführung die Elektrodenwendel einer weiteren Elektrodenleitung 5, bei der, ebenso wie bei der Ausführung nach Fig. 4A und 4B, in einer Funktions-Wendel 6 Magnetfluss-Erzeugungsmittel koradial eingepasst sind. Jedoch sind bei dieser Ausführung in zwei induktive Abschnitte L1a bzw. L1b der Funktions-Wendel 6 jeweils ein erster zusätzlicher Wendelabschnitt 7a mit einer Induktivität La, dem eine Kapazität Ca zugeordnet ist, und ein zweiter zusätzlicher Wendelabschnitt 7d mit einer Induktivität Lb, dem eine Kapazität Cb zugeordnet ist, eingefügt. Hierdurch werden zwei separat auf verschiedene Frequenzen abstimmbare Magnetflusssperren In der Elektrodenleitung gebildet. Unabhängig davon kann die über die Länge eines Funktions-Leiters verteilte Anordnung einzelner Magnetfluss-Erzeugungsmittel Vorteile hinsichtlich der mechanischen Eigenschaften der Implantatserbringer, insbesondere eine höhere Biegsamkeit.

Fig. 6 zeigt als weitere Ausführung eine Elektrodenleitung 8 mit einem gewendelten Funktions-Leiter 9 und einem koaxial hierzu angeordneten, den Funktions-Leiter 9 umgebenden weiteren Wendelabschnitt 10 mit einer Induktivität L und einer zugeordneten Kapazität C. Im Übrigen kann bei der koaxialen Ausführung die Wickelrichtung der Wendel der Magnetfluss-Erzeugungsmittel auch zur Wickelrichtung des Funktions-Leiters entgegengesetzt sein.

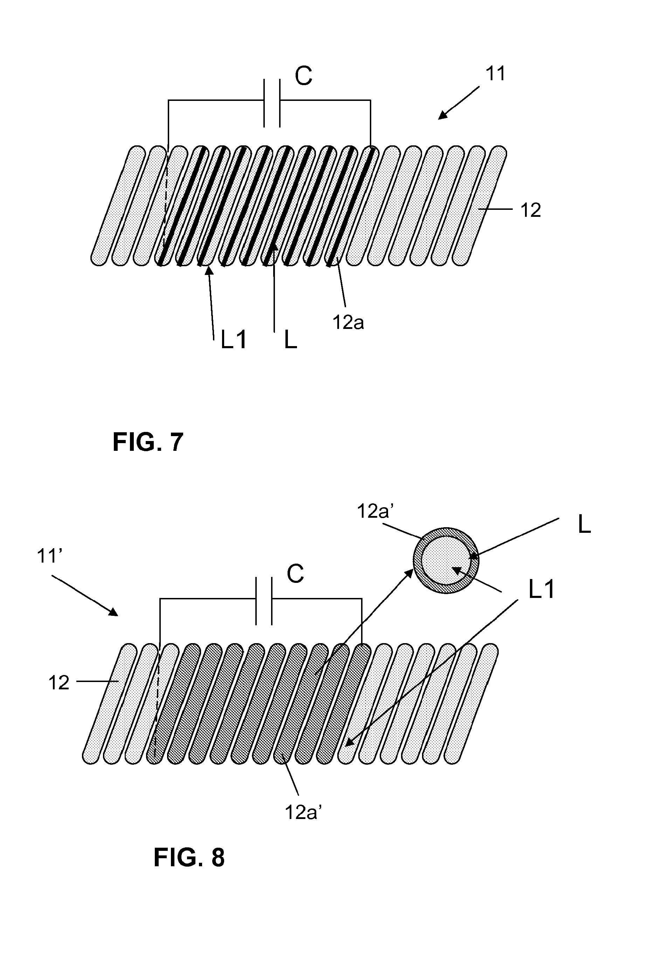

Fig. 7 zeigt als weitere, konstruktiv besonders einfache Ausführung der Erfindung eine Elektrodenleitung 11, bei der der Funktions-Leiter 12 in einem Abschnitt mit einer Induktivität L1 mit einer Beschichtung 12a versehen ist, die die Induktivität L eines mit der Kapazität C gebildeten Schwingkreises als Magnetfluss-Erzeugungsmittel realisiert. Die Beschichtung ist hier auf den Funktions-Leiter in Form zweier halbzylindrischer Abschnitte aufgebracht.

Fig. 8 zeigt als Abwandlung eine Elektrodenleitung 11 ', bei der der Funktions-Leiter 12 im relevanten Abschnitt mit einer den gesamten Leiterumfang umschließenden Beschichtung 12a'versehen ist.

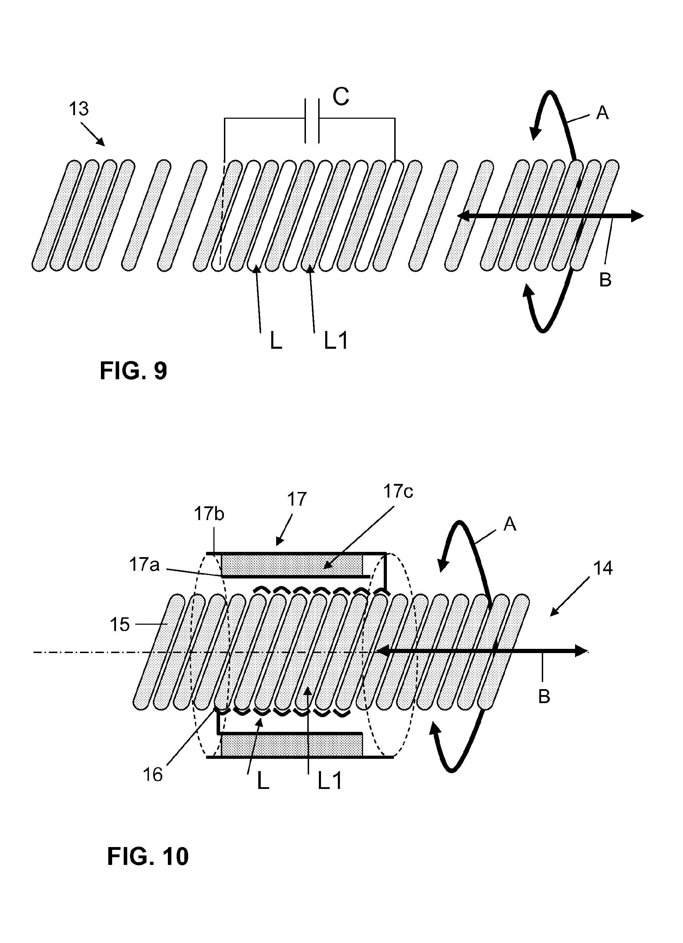

In Fig. 9 ist als weitere Ausführung schematisch der Innenaufbau einer Elektrodenleitung 13 gezeigt wobei die Pfeile A, B eine mechanische Beweglichkeit der Magnetfluss-Erzeugungsmittel bezüglich des Funktions-Leiters bzw., genauer gesagt, der jeweils diesen Elementen zugehörigen Wendelabschnitte, symbolisieren. Auf diese Weise kann mechanisch ein Steigungsgeber realisiert werden. Insbesondere kann die Beweglichkeit in Art einer Schraube-Mutter-Beziehung zwischen den Wendelabschnitten realisiert sein, in dem das induktive Element L wie eine Mutter in den Funktionsleiter L1 eingreift. Das kapazitive Element ist nur symbolhaft dargestellt. Vorteilhaft ist es wenn dabei der Funktionsleiter L1 gegenüber L bewegt wird.

Fig. 10 zeigt als Abwandlung dieser Ausführung eine Elektrodenleitung 14, bei der der Funktionsleiter 15 wie eine Schraube in einem Gewinde einer Mutter beweglich ist. Dabei wird das Innengewinde durch eine leitfähige Struktur 16 gebildet, wobei die Windungen jeweils seitlich zueinander keinen Kontakt haben. Wie bei der Ausführung nach Fig. 4B ist ein den relevanten Abschnitt des Funktions-Leiters 15 ummantelnder Schwingkreis-Kondensator 17 mit einem Innenzylinder 17a, einem Außenzylinder 17b und einem Dielektrikum 17c vorgesehen, der an das induktive Element 16 des Schwingkreises des Funktions-Leiters angeschlossen ist.

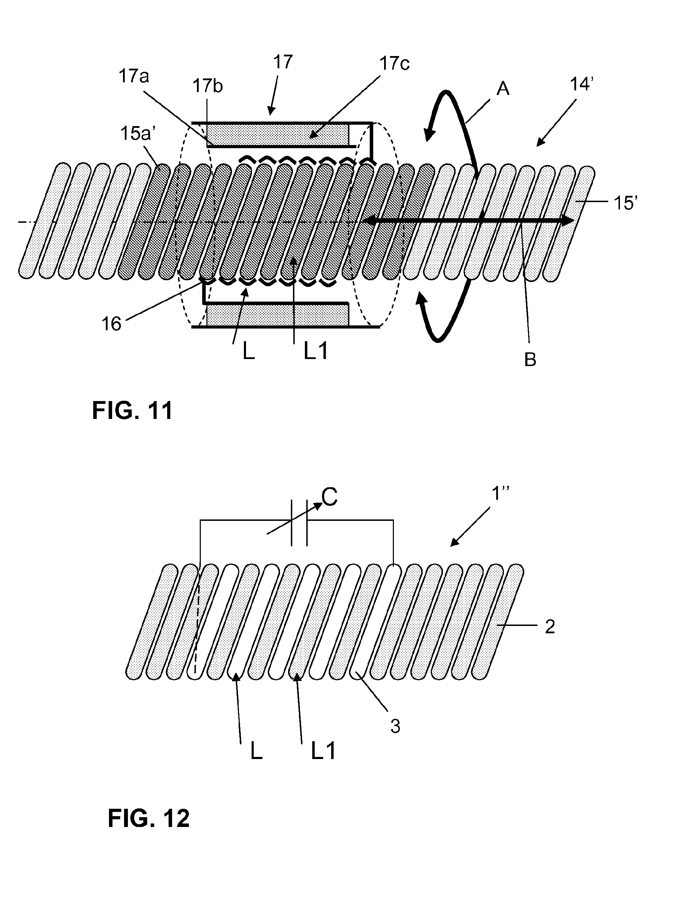

Fig. 11 zeigt als weitere Modifikation das Innere einer Elektrodenleitung 14', bei der der Funktions-Leiter 15 im Schraube-Mutter-Translationsabschnitt als elektrisch hoch leitfähiger Wendelabschnitt 15a'ausgeführt ist wie der "Gewindeabschnitt" 16 der Magnetfluss-Erzeugungsmittel. Die Leitfähigkeit des vorstehend als hoch leitfähig bezeichneten Bereichs sollte bevorzugt mehr als doppelt so hoch sein wie diejenige des Funktions-Leiters, also der eigentlichen Elektrodenwendel. Alternativ kann auch der Funktionsleiter L selbst hochleitend sein.

Fig. 12 zeigt in einer an Fig. 4A orientierten Prinzipskizze als weitere Ausführung eine Elektrodenleitung 1", die die gleichen konstruktiven Merkmale wie die Elektrodenleitung 1 nach Fig. 4A hat. Jedoch ist der Kondensator C des LC-Schwingkreises (der Magnetfluss-Erzeugungsmittel) hier in seiner Kapazität, etwa spannungsgesteuert, einstellbar, um die Wirkfrequenz der mit den Magnetfluss-Erzeugungsmitteln bewirkten Störsignalreduzierung einzustellen. In einer bevorzugten Ausführung erfolgt die Justierung, d. h. das Trimmen des Kondensators C, automatisch in Abhängigkeit von der Störfrequenz.

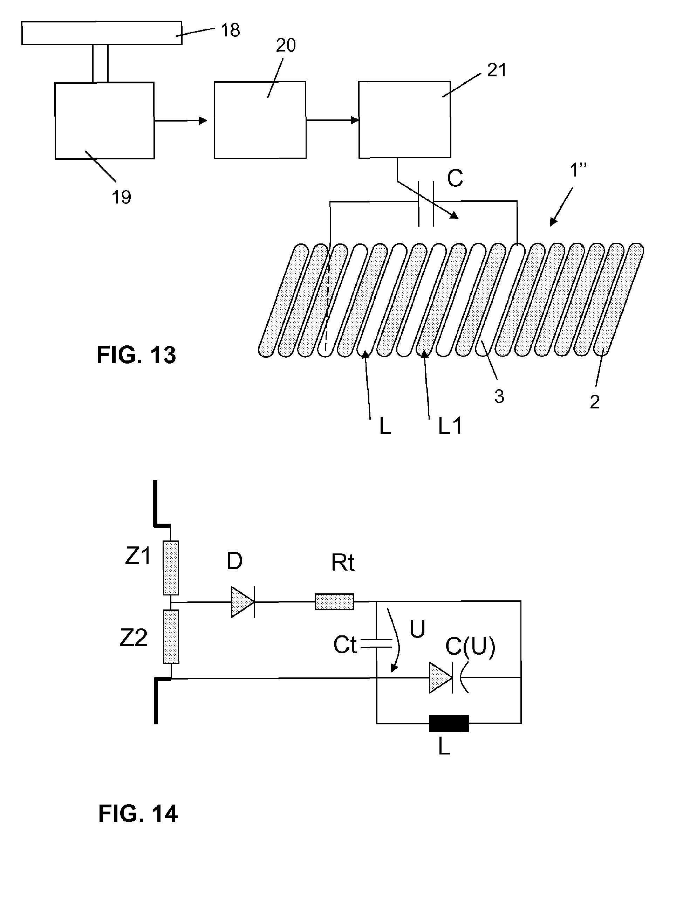

Fig. 13 zeigt eine konkrete Implementierung dieser automatischen Justierung der Magnetfluss-Erzeugungsmittel am Beispiel der Elektrodenleitung 1" aus Fig. 12. Die gezeigte Anordnung umfasst einen Feldsensor 18 (etwa einen Dipol), einen mit diesem eingangsseitig verbundenen Frequenz-Spannungs-Wandler 19, einen mit jenem eingangsseitig verbundenen Demodulator 20 und schließlich ein Tiefpassfilter 21, mit dessen Ausgangsignal die Kapazität des Kondensators C gesteuert wird. Den Frequenz-Spannungswandler realisiert speziell etwa ein Spannungsteiler aus zwei Impedanzen, etwa einem Widerstand und einer Induktivität. Der Demodulator 20 kann durch eine Diode realisiert sein, und eine sinnvolle Realisierung des Tiefpassfilters ist ein RC-Glied. Der spannungsabhängige Kondensator C selbst kann etwa eine Kapazitätsdiode (Varicap, Varactor) sein. Eine entsprechende Ausführung zeigt Fig. 14, wobei die Impedanzen Z1, Z2 den Frequenz-Spannungswandler 19, die Diode D den Demodulator 20 und Rt, Ct den Tiefpass 21 aus Fig. 13 realisieren. Die Kennlinie der spannungsabhängigen Kapazität wird so gewählt, dass die Wirkfrequenz genau der mit dem Feldsensor 18 erfassten Frequenzen des äußeren Störfeldes folgt. Alternativ kann auch der Frequenz/Spannungs-Wandler mit einer entsprechend kompensierenden Kennlinie ausgebildet sein.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

## Patentansprüche

1. Implantierbares Element mit einem langgestreckten Grundkörper, einem sich in Längsrichtung des Grundkörpers erstreckenden oder diesen bildenden Funktions-Leiter, der zur Realisierung einer medizinischen Funktion des Elementes wirkt und einen induktiven Abschnitt hat, und Magnetfluss-Erzeugungsmitteln zur Erzeugung eines magnetischen Flusses in der Umgebung des Funktions-Leiters, insbesondere von dessen induktivem Abschnitt, die magnetisch derart mit dem Funktions-Leiter koppeln, dass dem bei einem Stromfluss durch den Funktions-Leiter erzeugten magnetischen Fluss entgegen gewirkt und somit die Stromflussdichte durch den Funktions-Leiter verringert wird.

2. Implantierbares Element mit einem langgestreckten Grundkörper, einem sich in Längsrichtung des Grundkörpers erstreckenden oder diesen bildenden Funktions-Leiter, der zur Realisierung einer medizinische Funktion des Elementes wirkt, und Mitteln zum Einführen oder Anbringen von Magnetfluss-Erzeugungsmitteln zur Erzeugung eines magnetischen Flusses in der Umgebung des Funktions-Leiters, insbesondere von dessen induktivem Abschnitt, die magnetisch derart mit dem Funktions-Leiter koppeln, dass dem bei einem Stromfluss durch den Funktions-Leiter erzeugten magnetischen Fluss entgegen gewirkt und somit die Stromflussdichte durch den Funktions-Leiter verringert wird.

3. Implantierbares Element nach Anspruch 1 oder 2, wobei die Magnetfluss-Erzeugungsmittel einen induktiv wirkenden Leiterabschnitt, der ohne Unterbrechung des Verlaufes des Funktions-Leiters in den Grundkörper eingefügt oder auf diesen aufgesetzt ist, und ein hieran zur Bildung eines LC-Gliedes angeschlossenes kapazitives Element aufweisen.

4. Implantierbares Element nacheinander Ansprüche 1 bis 3, ausgebildet als Elektrodenleitung mit mindestens einer Elektrode und einem Elektrodenanschlusskontakt an einem Ende der Leitung, wobei der Funktions-Leiter die Elektrode mit dem Elektrodenanschlusskontakt verbindet und mindestens einen gewendelten Abschnitt aufweist und die Stromfluss-Erzeugungsmittel einen koaxial oder koradial mit dem gewendelten Abschnitt des Funktions-Leiters gewendelten Flusserzeugungsabschnitt aufweisen.

5. Implantierbares Element nacheinander Ansprüche 1 bis 3, ausgebildet als Führungsdraht oder Katheter oder Gefäßstütze, wobei der Funktions-Leiter mindestens einen gewendelten Abschnitt aufweist und die Stromfluss-Erzeugungsmittel einen koaxial oder koradial mit dem gewendelten Abschnitt des Funktions-Leiters gewendelten Flusserzeugungsabschnitt aufweisen.

6. Implantierbares Element nach Anspruch 4 oder 5, wobei die Magnetfluss-Erzeugungsmittel mehrere längs des Funktions-Leiters hintereinander geschalteter Flusserzeugungsabschnitte umfassen.

7. Implantierbares Element nach einem der vorangehenden Ansprüche, wobei die Magnetfluss-Erzeugungsmittel ein in einen Innenraum des induktiven Abschnitts des Funktions-Leiters eingebettetes oder den induktiven Abschnitt mindestens teilweise umgebendes kapazitives Element aufweisen.

8. Implantierbares Element nach Anspruch 7, wobei das in den induktiven Abschnitt eingebettete oder ihn mindestens teilweise umgebende kapazitive Element durch zwei koaxiale Metallzylinder mit der elektrischen Füllung gebildet ist.

9. Implantierbares Element nach Anspruch 7 oder 8, wobei längs des Funktions-Leiters mehrere kapazitive Elemente hintereinander angeordnet sind.

10. Implantierbares Element nach einem der vorangehenden Ansprüche, wobei die Magnetfluss-Erzeugungsmittel mehrere Komponenten aufweisen, die durch Bemessung der jeweiligen Induktivität und/oder Kapazität auf verschiedene Frequenzen eines äußeren Feldes abgestimmt sind.

11. Implantierbares Element nach Anspruch 10, wobei die auf verschiedene Frequenzen abgestimmten Komponenten der Magnetfluss-Erzeugungsmittel durch längs des Funktions-Leiters hintereinander angeordnete induktive Abschnitte mit eingebetteten oder umgebenden kapazitiven Elementen gebildet sind, die verschiedene Werte der Induktivität bzw. Kapazität haben.

12. Implantierbares Element nach einem der vorangehenden Ansprüche, wobei der oder mindestens ein Flusserzeugungsabschnitt der Magnetfluss-Erzeugungsmittel als leitfähige Beschichtung des Funktions-Leiters, insbesondere von dessen induktiven Abschnitt, ausgebildet ist.

13. Implantierbares Element nach einer der vorangehenden Ansprüche, mit mechanischen Verstellmitteln zur Veränderung der Relativposition zwischen dem induktiven Abschnitt des Funktions-Leiters und dem oder einem Flusserzeugungsabschnitt der Magnetfluss-Erzeugungsmittel.

14. Implantierbares Element nach einem der vorangehenden Ansprüche, wobei die Magnetfluss-Erzeugungsmittel, insbesondere im implantierten Zustand des Elementes, auf eine vorbestimmte Wirkfrequenz einstellbar sind.

15. Elektronisches Implantat, insbesondere Herzschrittmacher oder implantierbarer Defibrillator, mit Anschlussmitteln, die zum Anschluss eines implantierbaren Elementes nach einem der vorangehenden Ansprüche angepasst sind, und/oder mit integrierten Magnetfluss-Erzeugungsmitteln zur Erzeugung eines magnetischen Flusses in der Umgebung des Funktions-Leiters, insbesondere von dessen induktivem Abschnitt, die magnetisch derart mit dem Funktions-Leiter koppeln, dass dem bei einem Stromfluss durch den Funktions-Leiter erzeugten magnetischen Fluss entgegen gewirkt und somit die Stromflussdichte durch den Funktions-Leiter verringert wird.
